# EUROPEAN PATENT APPLICATION

(11) **EP 3 708 096 A1**
(43) Date of publication of application: **16.09.2020**
(21) Application number: 20160880.9
(22) Date of filing: 04.03.2020
(51) Int. Cl.: A61B 17/70, A61F 2/44, A61F 2/46, A61F 2/30

(54) **INTERVERTEBRAL IMPLANT ASSEMBLY AND INSTRUMENTS THEREFOR**

(30) Priority: 04.03.2019 US 201962813251 P
(71) Applicant: K2M, Inc., Leesburg, VA 20175 (US)
(72) Inventor: MILZ, Brian D., FLORIDA, NY 10921 (US); SOSNOV, Zinoviy, FAIR LAWN, NJ 07410 (US); ALHEIDT, Thomas A., SUSSEX, NJ 07461 (US); RYAN, Anthony, YORKTOWN HEIGHTS, NY 10598 (US); GREENDYK, Steven, BUTLER, New Jersey 07405 (US)
(74) Representative: Regimbeau

(57) **Abstract**

A spinal fusion system comprising an interbody device includes a leading end, trailing end, and opposed bone contacting sides extending therebetween. The trailing end defines a threaded opening that extends toward the leading end. The system also includes a bone plate that includes inner and outer surfaces and defines a first bone screw opening and connection screw opening extending therethrough. The system further includes a connection screw that includes a head and a threaded shaft. The head is positioned within the connection screw opening of the bone plate and is rotatably connected thereto such that the connection screw is rotatable about a longitudinal axis thereof but is prohibited from translational movement relative to the bone plate. The threaded shaft extends from the connection screw opening and is configured for threaded engagement with the threaded opening of interbody device.

## Description

### BACKGROUND OF THE INVENTION

The spine is comprised of a plurality vertebrae that are cushioned by intervertebral discs. Such intervertebral discs can deteriorate or become damaged due to injury, disease, or extended wear which may result in significant back pain that limits a patient's mobility and quality of life. One method of treatment that has been widely utilized is spinal fusion, whereby an affected disc is partially or fully excised and vertebral bodies adjacent such disc are fused together through the use of interbody devices, such as spacers, cages, and the like.

The aforementioned devices are often packed with a bone growth promoting material, such as bone graft and/or any known combination of biologics. In addition, some interbody devices include porous surfaces that promote bone growth into the structure of the interbody device itself. However, it takes time for bone to grow into the interbody device and for bone to grow between adjacent vertebrae to fuse the same. In the meantime, interbody devices often include mechanical features that help maintain engagement between the interbody device and bone of the existing vertebrae.

In order to help stabilize the spine during fusion, plates and/or rods are commonly used in conjunction with the interbody device. Plates are typically connected to adjacent vertebrae via bone screws and are positioned so that they extend across the disc space that contains the interbody device. While such plates may help stabilize the vertebrae, the interbody device is still solely reliant on its mechanical features to maintain its position within the disc space, which may not be in and of itself sufficient to prevent movement which should be prevented to encourage bone growth. In addition, bone plates can introduce additional complications, such as screw back-out which can result in plate loosing and destabilization. Therefore, further improvements are desirable.

### BRIEF SUMMARY OF THE INVENTION

In one aspect of the present disclosure, a spinal fusion system includes an interbody device that includes a leading end, trailing end, and opposed bone contacting sides extending therebetween. The trailing end defines a threaded opening extending toward the leading end. The system also includes a bone plate that includes inner and outer surfaces and defines a first bone screw opening and connection screw opening extending therethrough. The system further includes a connection screw that includes a head and a threaded shaft. The head is positioned within the connection screw opening of the bone plate and is rotatably connected thereto such that the connection screw is rotatable about a longitudinal axis thereof but is prohibited from translational movement relative to the bone plate. The threaded shaft extends from the connection screw opening and is configured for threaded engagement with threaded opening of interbody device.

Additionally, the head of the connection screw may be snap-fit to the bone plate. The connection screw opening may include first and second sections thereof. The first section may be positioned closer to the outer surface than the second section and may have a cross-sectional dimension smaller than the second section such that a first shoulder is formed therebetween. The head of the connection screw may be at least partially positioned within the second section of the connection screw opening. The shoulder may prevent the connection screw from backing out of the connection screw opening. The head of the connection screw may include a plurality of flexible members that are movable radially inwardly from a first to a second position when the head is passed through the first section of the connection screw opening and then from the second to the first position when the flexible members are at least partially positioned within the second section. The flexible members may each include a flange extending radially outwardly therefrom.

Continuing with this aspect, the threaded shaft may include a right-handed outer thread, and the connection screw may define a threaded interior opening that includes a left-handed inner thread. The bone plate may include a second bone screw opening that extends therethrough. The first and second bone screw openings may be aligned with the connection screw opening along a longitudinal axis of the bone plate. The plate may further define a screw blocker opening positioned adjacent the bone screw opening, and the system may include a screw blocker rotatably positioned within the screw blocker opening. The screw blocker opening may define first and second grooves that extend along a length of the screw blocker opening, and the screw blocker may include a projection that is alternately engageable to the first and second grooves. The screw blocker may include a flexible arm and the projection may extend from the flexible arm. The flexible arm may be moveable inwardly when the screw blocker is rotated between the first and second grooves of the screw blocker opening.

In another aspect of the present disclosure, a bone plate assembly includes a bone plate that includes inner and outer surfaces and defines a first bone screw opening and first screw blocker opening. The first bone screw opening extends through the inner and outer surfaces. The first screw blocker opening is positioned adjacent the first bone screw opening and has first and second indentations circumferentially offset from each other about a longitudinal axis of the screw blocker opening. A first screw blocker is positioned within the first screw blocker opening and has a head and a body extending from the head. The body includes a projection extending radially outwardly therefrom and is alternately engageable with the first and second indentations of the screw blocker opening.

Additionally, the first and second indentations may each be semi-cylindrical grooves that extend along a portion of a length of the screw blocker opening. The screw blocker opening may include a first section that extends through the inner surface of the bone plate and may be conical and a second section that may be cylindrical. The indentations may be located within the second section. The body of the first screw blocker may have a post at a distal end thereof. The post may have a tool opening defined by a sidewall thereof. The sidewall may be radially expanded from a first configuration such that the first screw blocker is rotatable within the first screw blocker opening but is prohibited from translational movement relative to the bone plate. The screw blocker body may include a flexible arm that depends downwardly from the head and may be moveable inwardly as the screw blocker is rotated about a longitudinal axis thereof between the first and second indentations. The projection may extend from the flexible arm. The head of the first screw blocker may be asymmetric about a plane extending through the first screw blocker. The screw blocker opening may extend through a recessed portion of the bone plate such that the screw blocker head is positioned within the recessed portion and is moveable within the recess from an unblocked position to a blocked position in which a portion of the head is positioned over the bone screw opening. The assembly may further a second screw blocker. The bone plate may further define a second bone screw opening and second screw blocker opening adjacent the first bone screw opening. The second screw blocker may be positioned within the second screw blocker opening.

In a further aspect of the present disclosure, a method of spinal fusion of adjacent vertebrae of a mammalian subject includes connecting an interbody device to a first inner member of an insertion tool positioned within an outer member of the insertion tool; inserting the interbody device into an intervertebral disc space using the insertion tool; disconnecting the interbody device from the first inner member; removing the first inner member from the outer member; inserting a second inner member into the outer member; connecting a bone plate to the second inner member; inserting the bone plate into the mammalian subject adjacent the intervertebral disc space; and driving a bone screw through a bone screw opening of the bone plate and into a first vertebra.

Additionally, connecting the interbody device to the first inner member may include rotating the first inner member in a first direction, and connecting the bone plate to the second inner member may include rotating the second inner member in a second direction opposite the first direction. The method may further include connecting the bone plate to the interbody device while the interbody device is positioned within the disc space. Also, connecting the bone plate to the interbody device may include rotating the second inner member in the first direction. Connecting the bone plate to the interbody device may also include threadedly engaging a connection screw connected to the bone plate to the interbody device. Further, connecting the bone plate to the second inner member may include threadedly engaging a threaded tip of the second inner member to the connection screw.

Continuing with this aspect, the method may further include connecting an adapter to the outer member, connecting a slap hammer to the adapter, and removing the interbody device from the intervertebral disc space via the slap hammer. Connecting the adapter to the outer member may include sliding the adapter over a knob of the first inner member. Connecting the adapter to the outer member may include pushing and rotating a knob of the adapter so that a threaded shaft of the adapter threadedly engages a threaded opening of the outer member. The outer member may include an angled shaft and the inner member may include an elongate shaft, a threaded tip, and a flexible portion disposed between the elongate shaft and threaded tip. The method may also include inserting the first inner member into a bore of the outer member such that a shoulder between the threaded tip and flexible portion is positioned in a facing relationship with a shoulder within the bore of the outer member. Even further, the method may include rotating a screw blocker from a first position to a second position so that a head thereof is positioned over the bone screw.

In a still further aspect of the present disclosure, a method of spinal fusion of adjacent vertebrae of a mammalian subject includes: connecting an interbody device to an insertion tool; inserting the interbody device into an intervertebral disc space using the insertion tool; disconnecting the interbody device from the insertion tool; and connecting a bone plate assembly to a second inner member. The bone plate assembly has a connection screw rotatably connected thereto such that the connection screw is rotatable about a longitudinal axis thereof but is prohibited from translational movement relative to the bone plate. The method also includes inserting the bone plate assembly into the mammalian subject adjacent the intervertebral disc space; and threadedly connecting a threaded shaft of the bone plate assembly to the interbody device while the interbody device is positioned within the disc space.

Additionally, the threadedly connecting step may be performed using the insertion tool. Connecting the interbody device to the insertion tool may also include rotating a first inner member of the insertion tool in a first direction, and connecting the bone plate assembly to the insertion tool may include rotating a second inner member of the insertion tool in a second direction opposite the first direction. Connecting the bone plate assembly to the interbody device may also include rotating the second inner member in the first direction.

In an even further aspect of the present disclosure, an insertion and extraction system for an interbody device includes an insertion tool that includes an outer member and an inner member, and an adapter that includes a body that defines a hollow compartment therein. The hollow compartment is configured to receive a portion of the outer and inner members. The adapter is connectable to the outer member. The system also includes a slap hammer connected to the adapter that has a sliding weight and a bumper for being bumped by the sliding weight.

Additionally, the inner member may include a knob extending from a proximal end of the outer member. The hollow compartment may be configured to receive the proximal end of the outer member and the knob. The adapter may include a threaded opening extending therein, and the slap hammer may have a threaded projection engageable with the threaded opening of the adapter. The adapter may include a spring and threaded shaft. The spring and threaded shaft may be disposed within a transverse opening extending into the body of the adapter and communicating with the hollow compartment. The spring and threaded shaft may be arranged such that the spring biases a threaded end of the threaded shaft away from the hollow compartment. The body of the adapter may include a post and the transverse opening that extends through the post, and the adapter may include a knob disposed over a portion of the post and may be connected to the inner shaft such that rotating the knob rotates the threaded shaft. The threaded shaft may include a collar extending radially outwardly therefrom and the spring may be arranged such that its bias pushes against the collar. Also, pushing on the knob may overcomes the bias of the spring to translate the threaded shaft from a first position in which the threaded end is disposed within the post and a second position in which the threaded tip is positioned within the hollow cavity. The outer member may also define a transverse threaded opening extending therein. When the insertion tool is received within the hollow cavity of the adapter, the threaded end of the threaded shaft may align with the transverse threaded opening of the outer member so that threaded member can be moved into engagement with the threaded opening over the bias of the spring.

In yet a further aspect of the present disclosure, a method of extracting an interbody device from an intervertebral disc space includes the steps of: sliding an adapter over a handle of an inserter; actuating a knob of the adapter to dispose a shaft of the adapter within a bore of the inserter; coupling a slap hammer to the adapter, the slap hammer including a sliding weight and a guide rod; and sliding the sliding weight along the guide rod to exert a force on the handle in a direction opposite the implant so as to transfer such force to the implant to remove it from the intervertebral disc space.

Additionally, the method may include connecting the adapter to the inserter by threadedly engaging a threaded shaft of the adapter with the bore of the inserter which is a threaded bore. Also, actuating the knob of the adapter may further include pushing on the knob to overcome a spring biasing the threaded shaft, and turning the knob to threadedly engage a threaded end of the threaded shaft and the bore. The inserter may include an outer member and an inner member. The inner member may have a threaded tip, an elongate shaft, and a flexible portion disposed between the threaded tip and elongate shaft. The outer member may include the handle, and sliding the adapter over the handle of the inserter may also include sliding the adapter over a knob of the inner member. The slap hammer may further include a threaded projection extending from a distal end surface and along a longitudinal axis of the slap hammer, and coupling the slap hammer to the adapter may further include threading the threaded projection into a threaded bore at a proximal surface of the adapter. The threaded bore may extend into the adapter along a longitudinal axis thereof. The method may further include aligning indicia on the adapter with indicia on the inserter prior to sliding the adapter onto the inserter.

### BRIEF DESCRIPTION OF THE DRAWINGS

FIG. 1A is a perspective view of fusion assembly according to an embodiment of the present disclosure.
FIG. 1B is an exploded perspective view of the fusion assembly of FIG. 1A.
FIG. 2A is a rear perspective view of an intervertebral implant of the fusion assembly of FIG. 1A.
FIG. 2B is a front perspective view of the intervertebral implant of FIG. 2A.
FIG. 3A an exploded perspective view of a plate assembly of the fusion assembly of FIG. 1A.
FIG. 3B is a perspective view of the plate assembly of FIG. 3A.
FIG. 3C is a cross-sectional view of the plate assembly take along line C-C of FIG. 3A.
FIG. 4A is a perspective view of a screw blocker of the plate assembly of FIG. 3A.
FIG. 4B is a cross-sectional view of the screw blocker taken along line B-B of FIG. 4A.
FIGS. 4C and 4D are cross-sectional views of the screw blocker of FIG. 4A positioned within a plate of the plate assembly of FIG. 3A.
FIG. 4E is an enhanced view of a screw blocker opening within the plate of the plate assembly of FIG. 3A.
FIG. 4F is an enhanced view of the screw blocker of FIG. 4A in a first position within the plate of the plate assembly of FIG. 3A.
FIG. 4G is a cutaway view of the screw blocker and plate of FIG. 4F.
FIG. 4H is an enhanced view of the screw blocker of FIG. 4A in a second position within the plate of the plate assembly of FIG. 3A.
FIG. 4I is cutaway view of the screw blocker and plate of FIG. 4H.
FIG. 5A is a perspective view of a connecting screw of the plate assembly of FIG. 3A.
FIG. 5B is a cross-sectional view of the connecting screw of FIG. 5A taken along a midline thereof.
FIG. 5C is a cross-sectional view of the connecting screw of FIG. 5A taken along a midline thereof and within the plate of the plate assembly of FIG. 3A.
FIG. 6A is a perspective view of the fusion assembly of FIG. 1A.
FIG. 6B is partial cross-sectional view taken along line B-B of FIG. 6A.
FIG. 7A is a perspective view of a plate assembly according to another embodiment of the present disclosure.
FIG. 7B is a side view of a fusion assembly according to another embodiment of the disclosure including the plate assembly of FIG. 7A.
FIG. 8 is a perspective view of an insertion/extraction system according to an embodiment of the present disclosure.
FIG. 9A is a perspective view of an implant insertion configuration of the insertion/extraction system of FIG. 8.
FIG. 9B is a perspective view of an outer member of the insertion/extraction system of FIG. 8.
FIG. 9C is a perspective view of an inner member of the insertion/extraction system of FIG. 8.
FIG. 9D is an enhanced cutaway view of a distal end of the outer member of FIG. 9B.
FIG. 9E is an enhanced cutaway view of a proximal end of the outer member of FIG. 9B.
FIG. 9F is an enhanced cross-sectional view of the inner member of FIG. 9C taken along a midline thereof.
FIG. 9G is an enhanced cross-sectional view of an inner member according to another embodiment of the disclosure taken along a midline thereof.
FIG. 9H is an enhanced view of a proximal end of the insertion/extraction system of FIG. 8.
FIG. 9I is an enhanced cross-sectional view of the proximal end of FIG. 9G connected to the intervertebral implant of FIG. 2A taken along a midline thereof.
FIG. 10A is a perspective view of an adapter of the insertion/extraction system of FIG. 8.
FIG. 10B is an exploded view of the adapter of FIG. 10A.
FIG. 10C is a perspective view of the adapter being connected to the insertion configuration of the insertion/extraction system of FIG. 9A.
FIGS. 10D and 10E are cross-section views of the adapter in respective first and second configurations.
FIG. 11 is a perspective view of a slap hammer of the insertion/extraction system of FIG. 8.

### DETAILED DESCRIPTION

When referring to specific directions in the following discussion of certain devices, it should be understood that such directions are described with regard to the device's orientation and position during exemplary application to the human body. Thus, as used herein, the term "proximal" means closer to the operator or in a direction toward the operator, and the term "distal" means more distant from the operator or in a direction away from the operator. The term "anterior" means towards the front part of the body or the face, and the term "posterior" means toward the back of the body. The term "medial" means toward the midline of the body, and the term "lateral" means away from the midline of the body. The term "inferior" means toward the feet of the body, and the term "superior" means toward the head of the body. Also, as used herein, the terms "about," "generally" and "substantially" are intended to mean that slight deviations from absolute are included within the scope of the term so modified.

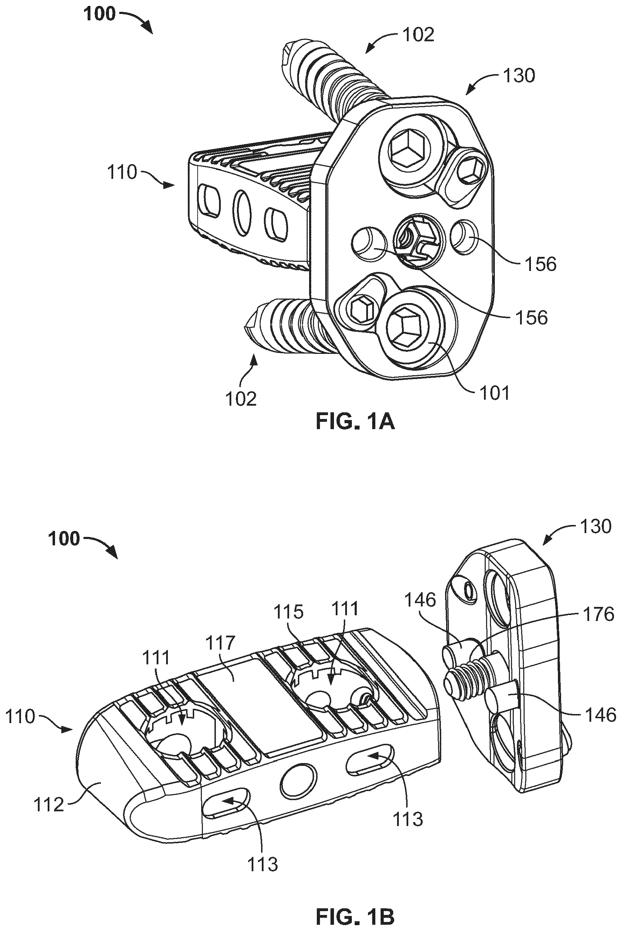

FIGs. 1A-6B depict a fusion assembly or system 100 according to an embodiment of the present disclosure. Fusion assembly 100 generally includes an intervertebral implant or interbody device 110 and a vertebral plate assembly 130.

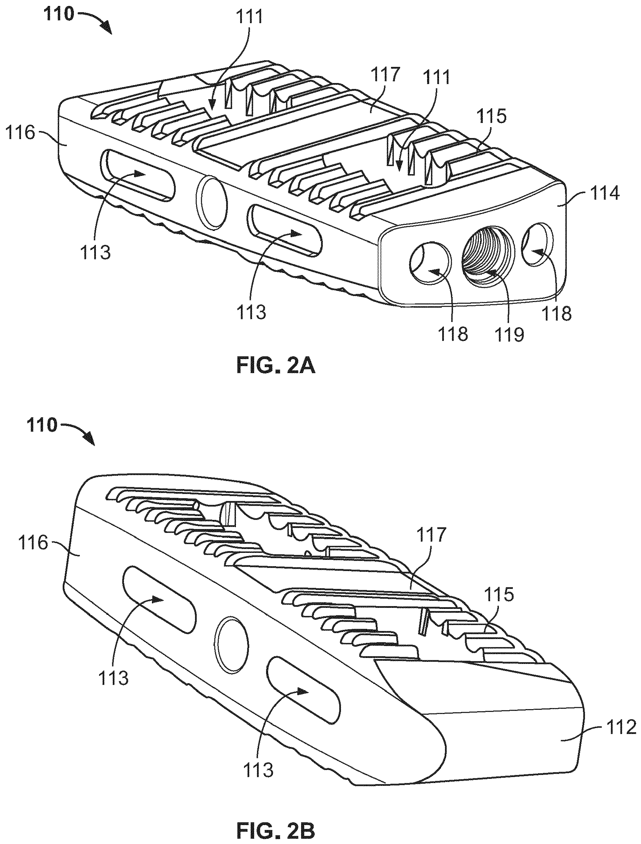

Interbody device 110, as depicted in FIGs. 2A and 2B, is particularly suited for a lateral approach. However, while interbody device 110 is described herein as being configured for a lateral approach, it is contemplated that other interbody devices may be used in fusion assembly 100, such as interbody devices configured for an anterior approach, for example. Exemplary lateral and anterior interbody devices that can be used in fusion assembly can be found in U.S. Patent No. 10,182,923, the disclosure of which is hereby incorporated by reference herein in in its entirety.

Interbody device 110 includes leading and trailing ends 112, 114. Leading end 112 has a rounded, wedge nose to facilitate insertion into an intervertebral space, as shown in FIG. 2B. Trailing end 114 defines a threaded opening 119 extending therein and extending toward leading end 112, as shown in FIG. 2A. Threaded opening 119 includes a right-handed thread and, in the embodiment depicted, communicates with a vertical graft window 111 closest to trailing end 114. However, in other embodiments, threaded opening 119 may terminate before reaching graft window 111 such that threaded opening 119 is a blind opening. Trailing end 114 also defines smooth bores/openings 118 that extend partially into trailing end 114 such that smooth bores 118 are blind openings. Smooth bores 118 flank threaded opening 119 and are aligned with threaded opening 119 in a mediolateral direction. However, in some embodiments, smooth bores 119 may be positioned such that axes thereof are offset superiorly - inferiorly relative to the axis of threaded opening 119.

Interbody device 110 also includes upper and lower bone contacting sides and lateral sidewalls 116 that extend between the leading and trailing ends 112, 114. Vertical graft windows 111 extend in a superior-inferior direction entirely through interbody device 110 and through the upper and lower bone contacting sides. Horizontal graft windows or lateral windows 113 extend in the mediolateral direction entirely through interbody device 110 and through lateral sidewalls 116. In the particular embodiment depicted, there are two vertical and two horizontal graft windows 111, 113. However, more or less of the vertical and horizontal graft windows 111, 113 is contemplated, such as one or more than two of each graft window 111, 113. Horizontal graft windows 113 intersect and are in communication with vertical graft windows 111 so that bone can grow vertically and horizontally through interbody device 110.

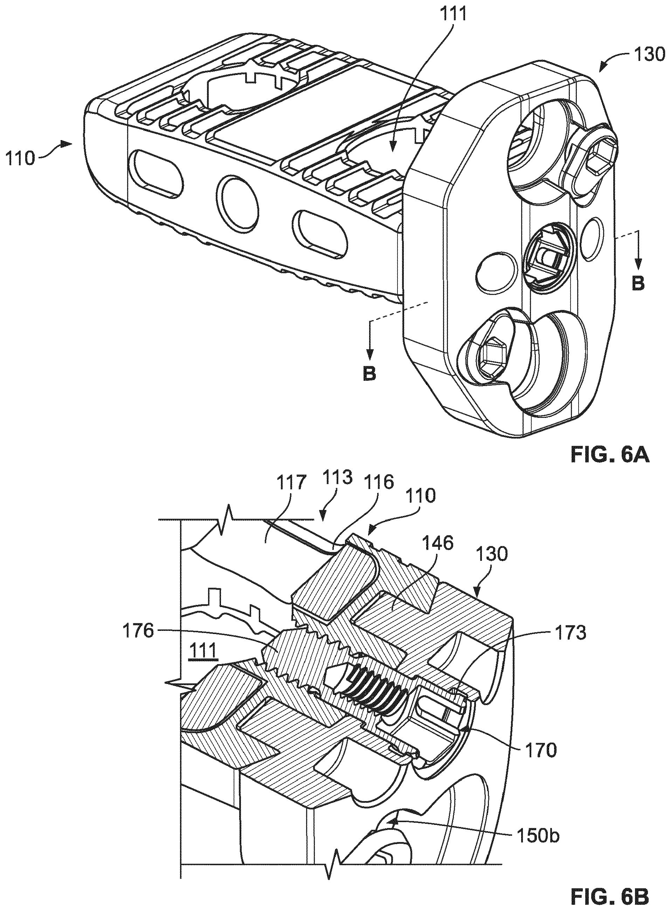

To further facilitate bone growth, interbody device 110 includes a porous structure 117 that forms at least a portion of upper and lower bone contacting sides. In addition, porous structure 117 defines at least a portion of a perimeter of vertical and horizontal graft windows 111, 113, as best shown in FIG. 6B. Porous structure 117 has a porosity that facilitates bone growth. For example, porous structure 117 may have an average pore diameter of 100 to 1000 microns with a 30-80% porosity, but preferably a porosity of 55% to 65%. In addition, a depth of porous structure 117 may be 500 to 4500 microns, but preferably 500 to 1500 microns. Interbody device 110 also includes a solid, non-porous frame that helps provide strength to interbody device 110. In particular, lateral sidewalls 116, trailing end 114, and leading end 112 comprise the solid frame and are thus made of a solid material. The solid frame and porous structure 117 can be made from any biocompatible metal, such as titanium and alloys thereof, and any biocompatible polymer, such as polyether ether ketone ("PEEK"), for example.

In addition to the solid frame, interbody device 110 also includes a plurality of solid serrations or teeth 115 that extend from the bone contacting sides. Such serrations 115 are configured to engage vertebral bodies to help limit movement of interbody device 110 while disposed within a disc space. In this regard, serrations 115 are made of a solid material, such as the same solid material that comprises the solid frame, to help provide sufficient strength to engage the bone and retain interbody device 110 relative to the vertebrae. However, in order to help maximize the volume of porous structure 117, such serrations 115 are embedded in the porous structure 117 and extend therefrom. Such embedment can be achieved through an additive layer manufacturing ("ALM") process, such as 3D printing, so that no separate connection mechanism is necessary to bring together any of the components of interbody device 110. In some examples, ALM processes are powder-bed based and involve one or more of selective laser sintering (SLS), selective laser melting (SLM), and electron beam melting (EBM), as disclosed in U.S. Pat. Nos. 7,537,664; 8,728,387; 9,180,010; and 9,456,901 as well as U.S. Patent Publication No. 2006/0147332, each of which is hereby incorporated by reference in their entireties herein. Other methods of ALM, which can be used to form the herein described implants, include stereolithography (SLA), fused deposition modeling (FDM), and continuous liquid interface production (CLIP).

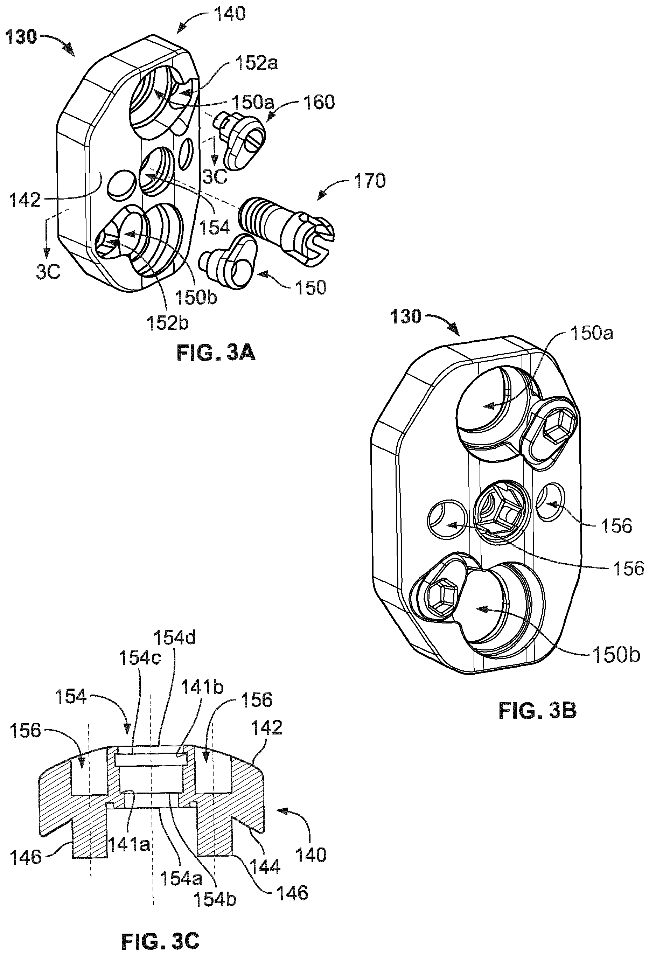

Vertebral plate assembly 130, as depicted in FIGs. 3A and 3B, generally includes a bone plate 140, screw blockers 160, and a connection screw 170. Plate 140 has a first side or bone contacting side and a second side or tool engagement side. The bone contacting side has a bone contacting surface or inner surface 144 that is concavely curved while the tool engagement side includes an outer surface 142 that is convexly curved so as to help conform plate 140 to the rounded outer surface of a vertebral body. The bone contacting side includes bosses or cylindrical projections 146 that extend from inner surface 144.

Plate 140 defines a plurality of openings that extend into and/or through plate 140 from the tool engagement side to the bone contacting side. Such openings include bone screw openings 150a-b, screw blocker openings 152a-b, connection screw opening 154, and smooth bores/openings 156. Bone screw openings 150a-b extend through outer and inner surfaces 142, 144 and are configured to receive a bone screw 102 therein such that a head 101 of bone screw 102 is positioned beneath outer surface, as shown in FIG. 1A.

Connection opening 154 is positioned between bone screw openings 150a-b and is aligned with bone screw openings 150a-b along a longitudinal axis of plate 140. However, in some embodiments, first bone screw opening 150a may be offset medially from connection opening while second bone screw opening 150b may be offset laterally, and *vice versa.* As shown in FIG. 3C, connection screw opening 154 is comprised of a plurality of sections 154a-d. A first section 154a extends through inner surface 144 while a fourth section 154d extends through outer surface 142. Second section 154b is adjacent to first section 154a, and third section 154c is adjacent to fourth section 154d and second section 154b. Second and third sections 154b-c, or intermediate sections, each have larger cross-sectional dimensions than that of first and fourth sections 154a, 154d. In addition, third section 154c has a larger cross-sectional dimension than second section 154b. These differences in cross-sectional dimensions form shoulders within connection screw opening 154. In particular, a first shoulder 141a is formed between first and second sections 154a-b, and a second shoulder 141b is formed between third and fourth sections 154c-d. First shoulder 141a faces toward outer surface 142 while second shoulder 141b faces toward inner surface 144. In the embodiment depicted, sections 141a-d are cylindrical. However, in some embodiments one or more of such sections 141a-d may be conical.

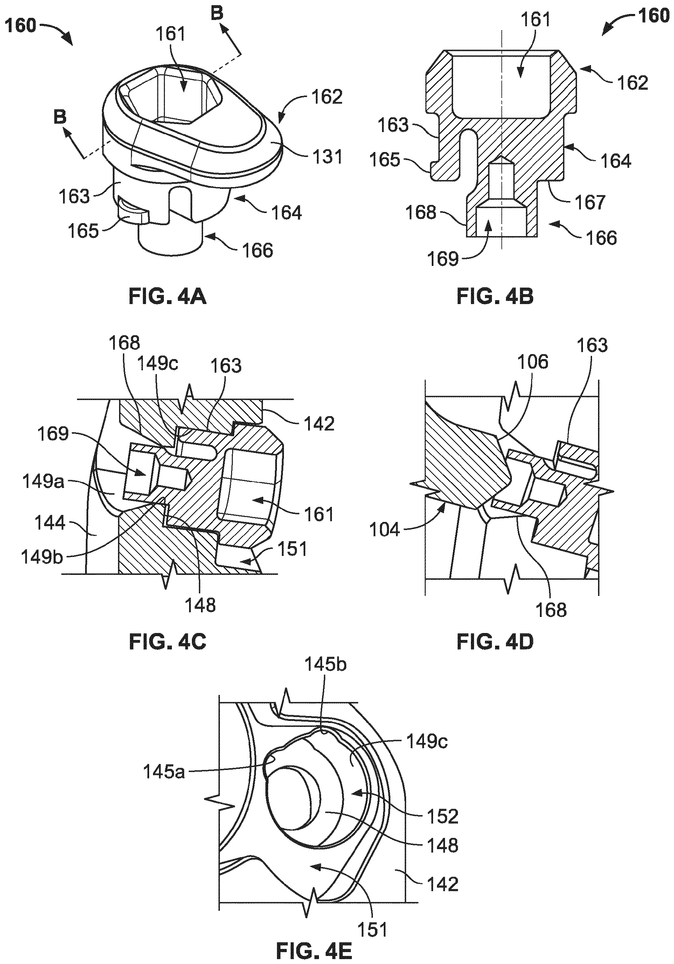

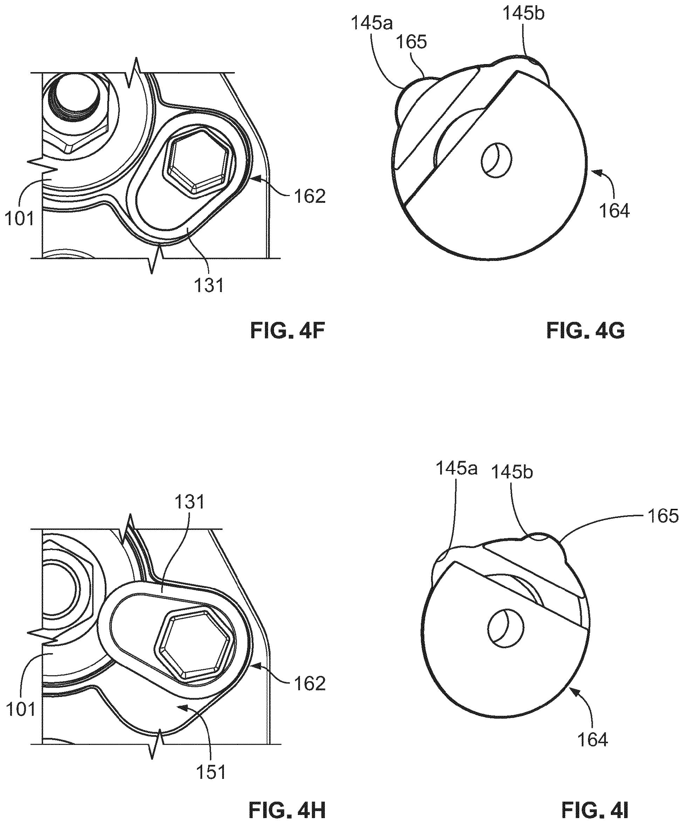

Screw blocker openings 152a-b are each positioned adjacent to an associated bone screw opening 150a-b and extend through an associated recessed region 151 of plate 140. Such recessed region 151, as best shown in FIGs. 4F and 4H, is shaped to accommodate a blocker head 162 of a screw blocker 160, as described in more detail below, such that screw blocker head 162 at least partially resides within recessed region 151. Screw blocker openings 152a-b each include first, second, and third sections 149a-c, as best shown in FIG. 4C. First section 149a extends through inner surface 144, and third section 149c is adjacent to recessed region 151. Second section 149b is intermediate first and third sections 149a, 149c. Second and third sections 149b-c are cylindrical while first section 149a is conical. In addition, third section 149c includes two semi-cylindrical indentations/grooves 145a-b that extend along the length of third section 149c and are circumferentially offset from each other, as best shown in FIG. 4E. Third section 149c also has a cross-sectional dimension greater than that of second section 149b which forms a shoulder 148 that faces toward outer surface 142 of plate 140. It should be understood that in some embodiments, blocker openings 152a-b may only include first and third sections 149a, 149c as first section 149a may have a conical taper that intersects with the third section 149c.

Smooth bores/openings 156 extend through outer surface 142 and partially into plate 140 such that smooth bores 156 are blind openings. Smooth bores 156 are aligned with connection screw opening 154 in a mediolateral direction. However, in some embodiments, smooth bores 156 may be positioned such that axes thereof are offset superiorly-inferiorly relative to an axis of connection screw opening 154. Also, as shown in FIG. 3C, each of bores 156 have an axis that coaligns with an axis of a corresponding boss 146.

Screw blockers 160, as depicted in FIGs. 4A and 4B, each include a blocker head or proximal end 162 and a blocker body extending from head 162. The blocker body includes an expansion post or distal end 166 and locking portion or intermediate portion 164. Blocker head 162 defines a tool engagement opening 161 and is asymmetrically shaped or tear drop shaped so that it forms a lobe 131 that extends radially further from a longitudinal axis of screw blocker 160 than any other portion of blocker head 162 on an opposite side of the longitudinal axis from lobe 131.

Intermediate portion 164 is positioned between blocker head 162 and expansion post 166. Intermediate portion 164 is substantially cylindrical and is cut such that a recess extends through intermediate portion 164 so as to form a flexible arm or tab 163 that is cantilevered to blocker head 162. Flexible arm 163 includes a lip or projection 165 that extends radially outwardly therefrom and is moveable in radially inwardly such that, when it is moved radially inwardly, flexible arm 163 is biased to its neutral position, as is shown.

Expansion post 166 extends distally from intermediate portion 164 and is substantially cylindrical. A tool opening 169 extends through post 166 toward and, in some embodiments, partially into intermediate portion 164. A post sidewall 168 extends about tool opening 169 and is deformable such that post 166 is expandable from a cylindrical shape to a conical shape, as described in more detail below.

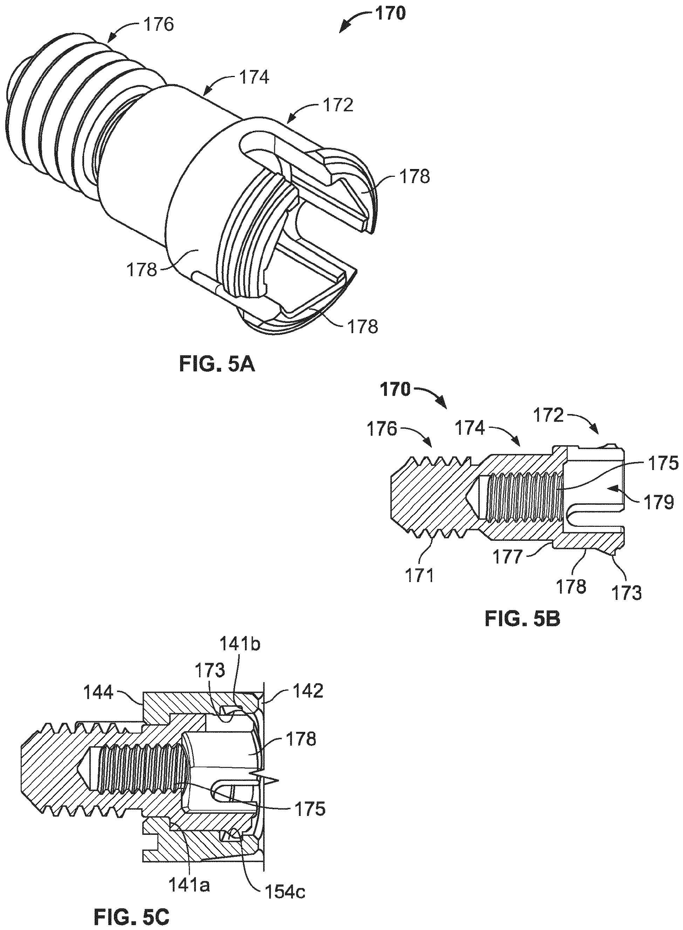

Connection screw 170, as depicted in FIGs. 5A and 5B, includes a head 172, distal shaft 176, and intermediate shaft 174. Head 172 includes a tool opening 179 and a plurality of flexible members or tabs 178 that are circumferentially positioned about tool opening 179. Such flexible members 178 are bendable radially inwardly toward a longitudinal axis of screw 170 but, when bent inwardly, are biased toward their neutral position, as is shown. Flexible members 178 each include a lip or flange 173 that extends radially outwardly therefrom and extends about a perimeter thereof.

Intermediate shaft 174 has a smooth outer surface that is substantially cylindrical and a threaded interior opening that includes a left-handed thread 175 that helically extends along the length of intermediate shaft 174. The threaded interior opening is in axial communication with the tool opening of head 179. An outer cross-sectional dimension of intermediate shaft 174 is smaller than an outer cross-sectional dimension of head 172 such that a distally facing shoulder 177 is formed therebetween. Distal shaft 176 extends from intermediate shaft 174 and includes a right-handed external thread 171 that helically extends along its length. Such thread 171 corresponds to that of threaded opening 119 of interbody device 110. A major diameter of thread 171 is greater than a major diameter of inner thread 175 of intermediate shaft.

When fusion assembly 100 is fully assembled, bosses 146 are positioned within respective ones of smooth bores 118 of interbody device 110, as best shown in FIG. 6B. In addition, connection screw 170 extends through connection screw opening 154 of plate 130 and into interbody device 110 such that external threads 171 of distal shaft 176 threadedly engage threaded opening 119 of interbody device 110. As shown in FIGs. 5C and 6B, head 172 of connection screw 170 is snap-fit to plate within connection screw opening 154. In this regard, when being loaded into plate 130, flexible members 178 are pushed inwardly via lips 173 contacting the narrower fourth section 154d of opening 154. When such lips 173 reach third section 154c of connection opening 154, flexible members 178 snap outwardly to their neutral position. In this position, first shoulder 141a of opening 141 abuts shoulder 177 of screw 170 to prevent further distal movement of screw 170, and second shoulder 141b of opening 141 abuts lips 173 to prevent further proximal movement of screw 170. However, screw 170 remains rotatable relative to plate 130 for threaded connection to interbody device 110.

Also in the fusion assembly 100, screw blockers 160 are positioned in their respective blocker openings 152a-d. More particularly, as shown in FIG. 4C, expansion post 166 of each blocker 160 extends through second section 149b of blocker opening 152 and into first section 149a, intermediate portion 164 is positioned within third section 149c of opening 152, and head 162 is at least partially positioned within recessed region 151. Screw blockers 160 are connected to plate 140 via deformation of expansion post 166, which may be performed via a tool 104, as shown in FIG. 4D. When tool 104 is advanced into tool opening 169, a tapered end 106 of such tool 104 causes sidewall 168 to expand outwardly and plastically deform such that post 166 generally conforms to the conical shape of first section 149a of blocker opening 152. This deformation prevents each screw blocker 160 from being removed from their respective opening 152a-b, but allows for such blockers 160 to be rotated therein.

When screw blockers 160 are positioned within their respective openings 152a-b, such blockers 160 have first and second locked positions associated with indents 145a-b. In this regard, when blockers 160 are in a first locked or unblocked position, as shown in FIGs. 4F and 4G, head 162 is positioned within recessed region 151 of plate 140 so that lobe 131 resides in the recessed region 151 and does not cover a bone screw head 101. In addition, when in the first locked position, lip 165 of flexible arm 163 is positioned in first indentation 145a of blocker opening 152. When blockers 160 are in a second locked or blocked position, as shown in FIGs. 4H and 4I, lip 165 is positioned within second indent 145b and lobe 131 is moved at least partially out of recessed region 151 so that it covers a portion of bone screw 102 head to prevent bone screw 102 from backing out of plate 140. When transitioning from the first to the second position, or *vice versa,* an operator uses a tool/driver engaged to blocker head 162 via opening 161 to rotate blocker 160. Once a predefined torque is achieved, flexible arm is cammed inwardly so that blocker 160 can be rotated to the next position. Once lip 165 reaches the next indentation 145a or 145b, the bias of arm 163 snaps lip 165 into engagement with the other indentation 145a or 145b to lock blocker 160 in that position. This mechanism not only locks blocker 160 into a desired angular positions relative to bone screw 102, but it also provides tactile feedback to the operator. Also, it should be understood that connection screw 170 and/or screw blockers 160 may be preloaded into plate 140 prior to delivery to the operating theater so that the operator does not have to assemble the plate during the procedure.

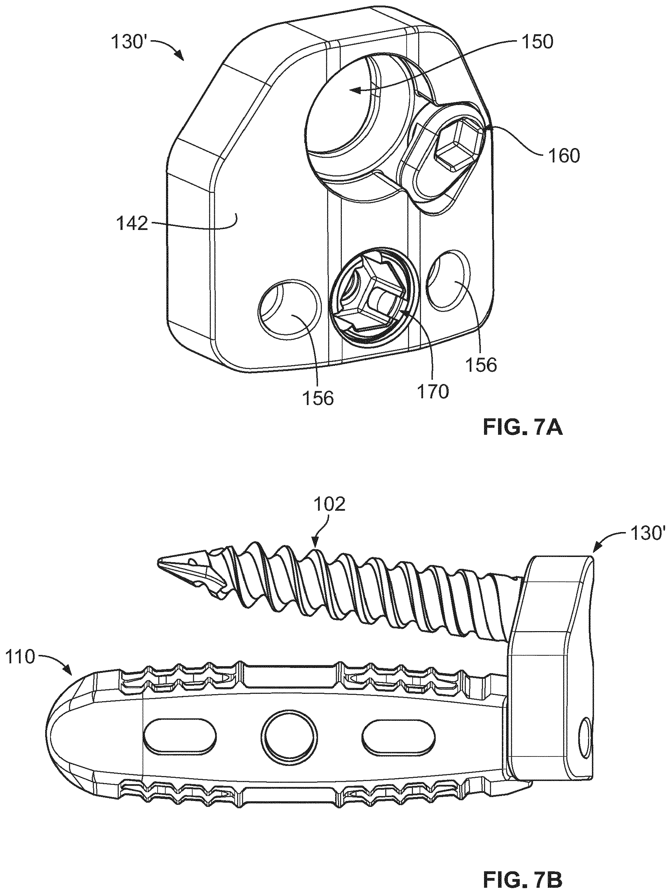

FIGs. 7A and 7B depicts an alternative plate assembly 130'. Plate assembly 130' differs from plate 130 in that it is configured to only be connected to one vertebra via a bone screw 102 rather than two. In this regard, plate assembly 130', while including connection screw 170 and bosses (not shown) for connection to implant 110, only includes one bone screw opening 150 and one associated screw blocker 160.

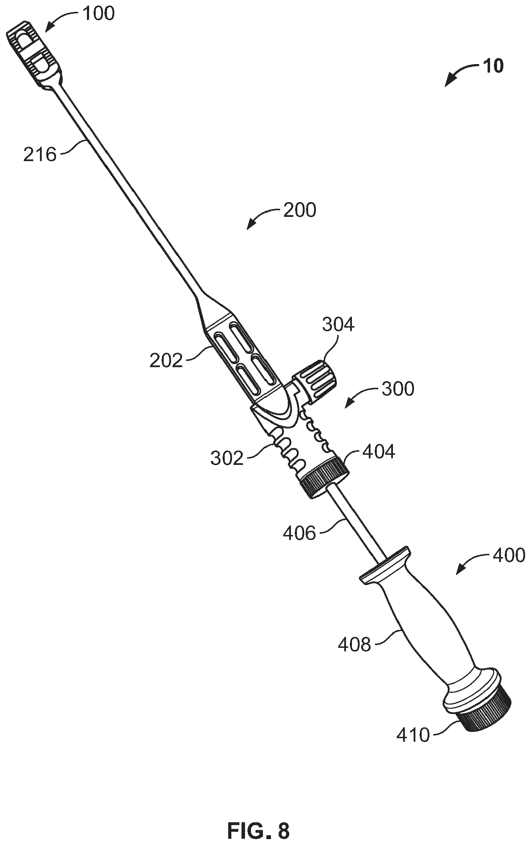

FIGs. 8-11 illustrate an insertion/extraction system 10 according to an embodiment of the present disclosure. Insertion/extraction system 10 is configured to insert interbody device 100 into an intervertebral disc space and extract interbody device 100 therefrom. System 10 is also configured to insert plate assembly 130 into the patient/mammalian subject and connect plate assembly 130 to interbody device 100 in-situ, as described in more detail below. System 10 includes an inserter/extractor 200, an adapter 300, and a slap hammer 400.

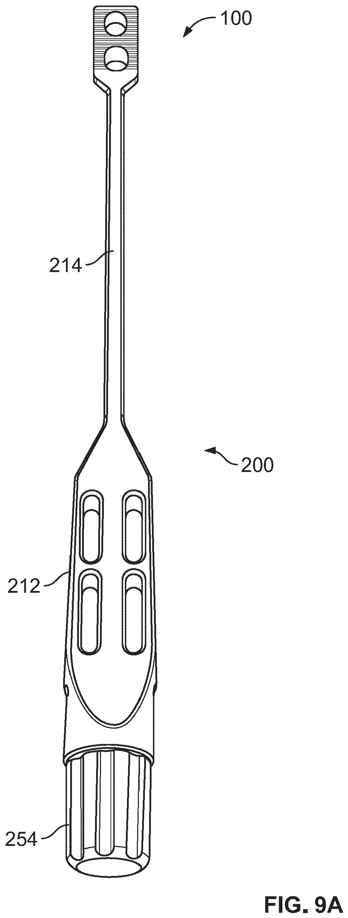

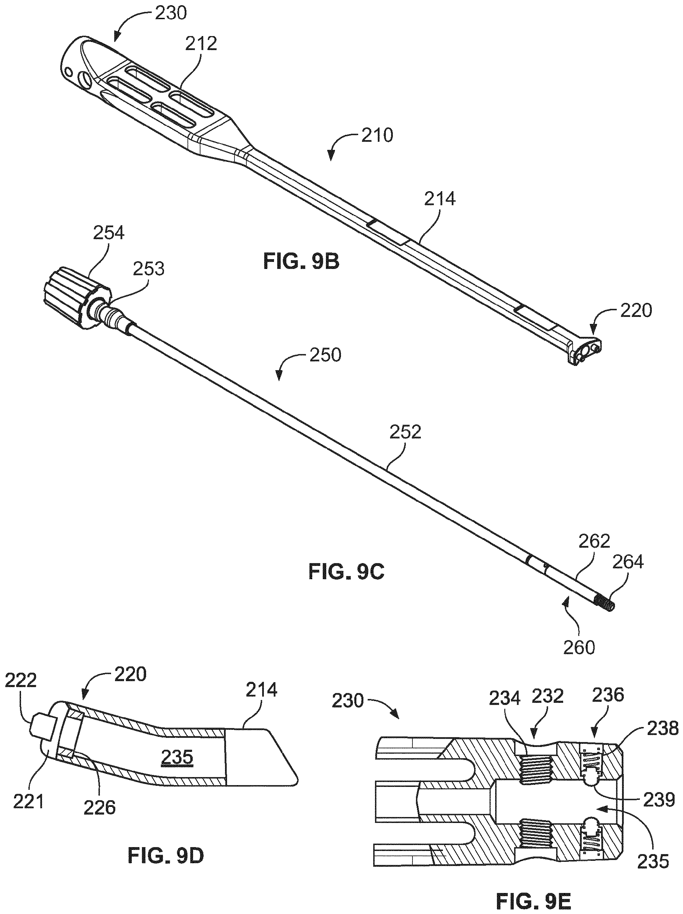

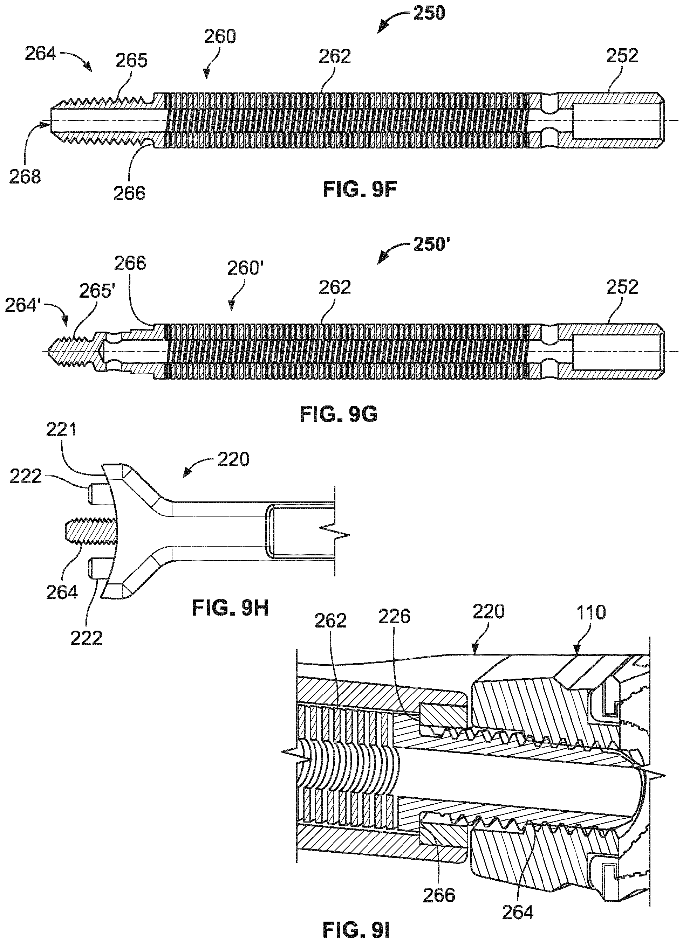

Inserter/extractor or insertion tool 200, as depicted in FIGs. 9A-9I, generally includes an outer member 210 and an inner member 250. Outer member 210 includes a handle 202, insertion end 220, adaptor end 230, and rigid shaft 214. Insertion end 220 is positioned at a distal end of rigid shaft 214 and is crescent shaped such that it has a concave distal facing surface 221, as best shown in FIG. 9H. Insertion end 220 includes a pair of bosses 222 extending distally therefrom for insertion into smooth bores 118, 156 of interbody device 110 and plate 140. Inserter/extractor 200, as shown, is an angled inserter/extractor such that insertion end 220 is angled relative to rigid shaft 214, as best shown in FIG. 9D. In other words, in some embodiments rigid shaft 214 may be bent just proximal of insertion end 220 to allow for interbody device 110 to be inserted into an intervertebral space from certain locations relative to such disc space, while in other embodiments rigid shaft 214 may not be bent so that interbody device 110 can be inserted from other locations relative to disc space.

Handle 212 is located at a proximal end of rigid shaft 214 and generally has more girth than rigid shaft 214 so as to easily fit in an operator's hand. Adaptor end 230 is located at a proximal end of handle 212 and includes threaded openings 232 and spring-ball mechanisms 236. Spring-ball mechanisms 236 each include a spring 238 and ball bearing 239 housed in adaptor end 230 and are positioned proximal of threaded openings 232, although in some embodiments this may be reversed. Spring-ball mechanisms 236, as well as threaded openings 232, are positioned at opposite sides of a longitudinal axis of inserter/extractor 200. A longitudinal bore 235 extends entirely through outer member 210 along the longitudinal axis thereof from adapter end 230 to insertion end 220 so that such bore 235 extends through concave surface 221, even where outer member 210 is angled. However, bore 235 abruptly narrows in dimension just proximal of concave surface 221 of insertion end 220. In other words, bore 235 has a first cross-sectional dimension greater than a second cross-sectional dimension of bore 235. Bore 235 has the second cross-sectional dimension at a distal terminal end thereof where bore 235 extends through concave surface 221. This difference in cross-section forms a shoulder 226, as best shown in FIGs. 9D and 9I. Ball bearings 239 of spring-ball mechanisms 236 communicate with bore 235 at a proximal end thereof, and threaded openings 232 extend from an exterior of adapter end 230 toward bore.

Inner member or first inner member 250 generally includes an elongate shaft 252, knob 254, and a connection end 260. A longitudinal bore 268 may extend through connection end 260 and into elongate shaft such that inner member 250 can receive a guidewire, as best shown in FIG. 9F. Knob 254 is located at a proximal end of elongate shaft 252 and is located adjacent a groove 253 on elongate shaft 214 that is configured to receive ball bearings 239 of the ball-spring mechanisms 236 of outer member 210 so as to form a ball-detent mechanism that allows inner member 250 to be removably connected to outer member 210. Connection end 260 is located at a distal end of elongate shaft 252 and includes a threaded tip 264 and a flexible portion 262, as best shown in FIG. 9F. Flexible portion 262 is more proximal than threaded tip 264 and may be a coil, spring, goose neck, or the like so that threaded tip 264 can be angled relative to elongate shaft 252. This allows inner member 250 to extend through bore 235 of outer member 210 even when outer member 210 is angled. Thus, in embodiments where outer member 210 is not angled, inner member 250 may not include flexible portion 262. Elongate shaft 252, including flexible member 262, has a larger cross-sectional dimension than a minor diameter of threaded tip 264, which forms a shoulder 266. Threaded tip 264 also has threads 265 with a first major diameter which is particularly configured for threaded connection with threaded opening 119 of interbody device 110.

A second inner member 250' is shown in FIG. 9G and includes an alternative threaded tip 260' that is particularly configured for threaded connection with threaded opening of connection screw 170. In this regard, inner member 260' has second major diameter smaller than that of inner member 260. Also, threads 265' of threaded tip 260' are left-handed threads whereas threads 265 of first inner member 260 are right-handed threads. However, inner member is otherwise similarly configured such that it may include a flexible portion and shoulder 266, as shown.

When inserter/extractor 200 is assembled, inner member 250 extends through bore 235 of outer member 210 so that threaded tip 264 of inner member 250 extends from insertion end 220 and is positioned between bosses 222, as best shown in FIG. 9H. Flexible portion 262 of inner member 250 allows inner member 250 to conform to the angled outer member 210. In addition, shoulder 226 of outer member 210 provides a distal limit for inner member 250 such that shoulder 266 of inner member 250 abuts shoulder 226 before the entirety of threaded tip 264 can extend from bore 235. Inner member 250 is removably connected via ball bearings 239 of spring-ball mechanisms 236 being positioned in groove 253 at the proximal end of elongate shaft 252. However, this allows knob 254 to rotate inner shaft 252. As such, interbody device 110 can be connected to inserter/extractor 200 by threadedly engaging threaded opening 119 via threaded tip 264 by rotating knob 254. This pulls interbody device 110 into engagement with concave surface 221 and onto bosses 222 so that such bosses 222 are each positioned within a corresponding smooth bore 118 of interbody device 110 thereby providing anti-rotation. In addition to drawing interbody device 110 onto insertion end 220, shoulders 226 and 266 are brought into firm contact. Such shoulder-to-shoulder contact helps protect threads 265 during insertion and flexible portion 262 during extraction of interbody device 110. In particular, the flexible construction of flexible portion 262 is less robust than a rigid counterpart and is, therefore, prone to damage when stressed. However, the shoulder-to-shoulder contact mentioned above helps ensure undue stresses are not applied to flexible portion 262 during extraction, as described in more detail below.

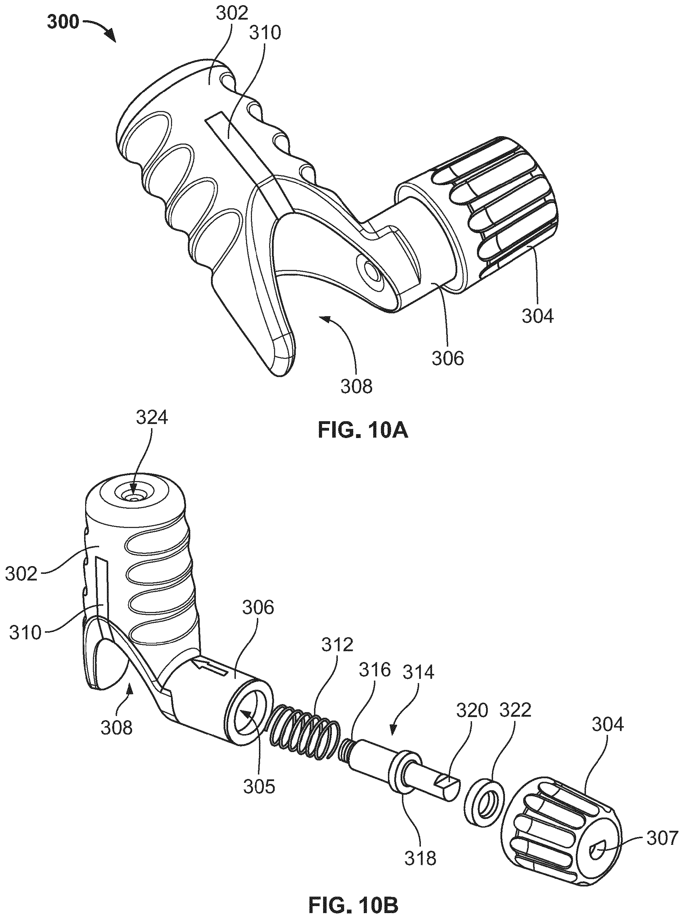

FIGs. 10A-10E illustrate an adapter 300 that can be coupled to inserter/extractor 200. Adapter 300 has a body 302 with a hollow compartment 308 therein that is configured to receive the proximal end of inserter/extractor 200, including knob 254. Adapter 300 also includes a spring 312, piston or threaded shaft 316, washer or bushing 322, and knob 304. A post 306 extends from body 302 and has a transverse opening 305 in communication with hollow compartment 326. In the embodiment shown, post 306 is substantially cylindrical. Post 306 is capped with knob 304. However, spring 312, threaded shaft 314, and bushing 322 are positioned within post 306 such that spring 312 bears on an annular collar 318 of threaded shaft 314 and an inner shoulder 303 of post 306. In addition, bushing 322 is positioned at one end of post opening 305 so that collar 318 is positioned between shoulder 303 and washer 322. Thus, spring 312 exerts a biasing force on shaft 314 that biases collar 318 toward bushing 322 and, consequently, also biases a threaded end 316 of shaft 314 away from hollow compartment 308. However, when this bias is overcome, threaded end 316 of shaft 314 can be moved into hollow compartment 308. Threaded shaft 316 also includes a projection 320 at the opposite end of shaft 314 from threaded end 316. In the embodiment shown, projection 320 has a "D" shape. In this regard, projection 320 couples shaft 314 to knob 304, as knob 304 has an opening 307 of corresponding size and shape to that of projection 320. Thus, the flat surface of the D-shaped projection 320 allows the shaft 314 to be engaged with knob 304 such that when knob 304 is rotated, shaft 314 will also rotate. The shape of projection 320 can be any shape, though, that allows for such rotation, such as star shaped, hex shaped, and the like. Thus, in other embodiments projection 320 may have a different shape.

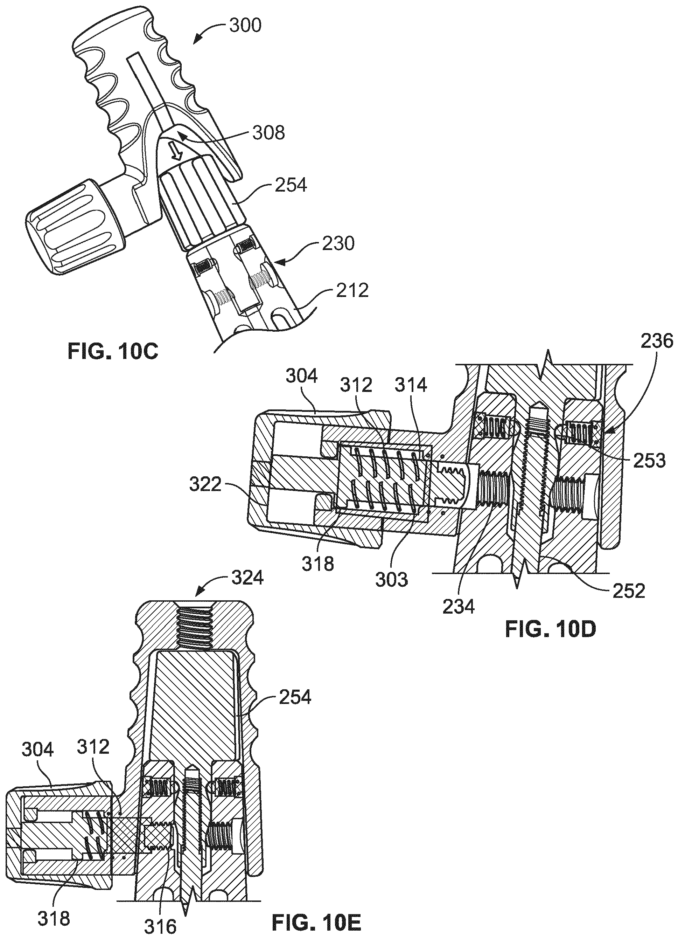

Adapter 300 can be connected to inserter/extractor 200. In this regard, adapter 300 is placed over knob 254 and adapter end 230 of outer member 210 such that the same is received within hollow compartment 308, as best shown in FIG. 10C. A laser mark or indicia 310 on handle 302 of adapter 300 and a corresponding laser mark on outer member 210 (not shown) allow for proper orientation of adapter 300. This helps ensure that the threaded shaft 264 aligns with one of threaded openings 232 in inserter/extractor 200. A corresponding taper of adapter end 230 of outer member 210 and hollow compartment 308 also allow threaded shaft 316 to align with threaded openings 232 in the proximal-distal direction. Once threaded shaft 314 is aligned with a threaded opening 232, knob 304 can be pushed inwardly along the longitudinal axis of post 306 and in a direction towards hollow compartment 308 to overcome the bias of spring 312. This causes knob 304 to advance threaded end 316 of shaft 314 into threaded opening 232 of inserter 200. Knob 304 is then rotated, while the push force is exerted on it, in order to engage threaded end 316 with threads 234 of threaded opening 232, as best shown in FIGs. 10D and 10E.

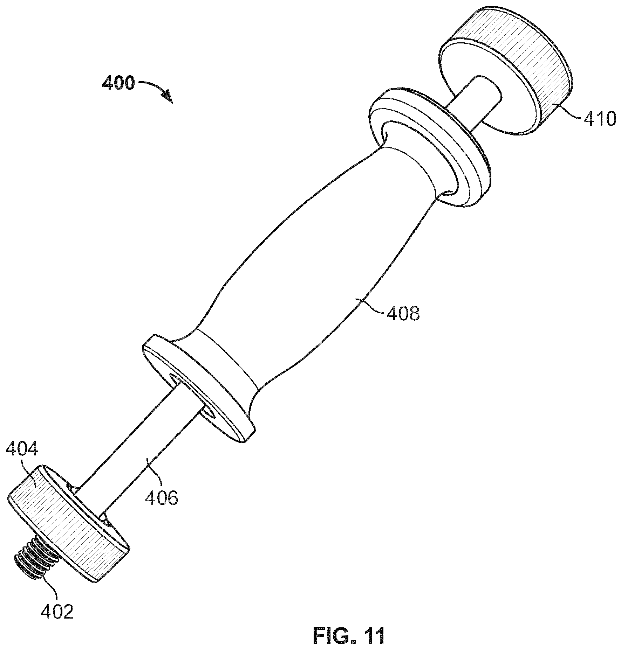

FIG. 11 depicts an embodiment of slap hammer 400. Slap hammer 400 includes a guide rod 406, sliding weight 408, and distal and proximal bumpers 404, 410. Guide rod 406 is substantially cylindrical. Sliding weight 408 has a throughbore (not shown) extending along its longitudinal axis, the diameter of which is at least slightly larger than that of guide rod 406 so that weight 408 can slide on rod 406. Guide rod 406 is positioned within the longitudinal throughbore of sliding weight 408 and extends past the proximal and distal end of sliding weight 408. Bumpers 404, 410 are positioned at each end of guide rod 406 so that sliding weight 408 can slide proximally and distally along guide rod 406, between bumper 404 and bumper 410. Thus, as sliding weight 408 bumps into bumpers 406, 408, a force is exerted on shaft 406. A threaded projection 402 extends distally from distal bumper 404 and along the longitudinal axis of shaft 406. Threaded projection 402 is configured to threadedly connect with a threaded opening 324 of adapter so that force caused by bumping weight 408 into proximal bumper 410 is transferred through adapter 300 when connected to slap hammer 400.

In a method of implanting fusion assembly 100, an operator accesses a target intervertebral disc between adjacent vertebrae and approaches such disc via a desired approach, such as a lateral or anterior approach, for example. The intervertebral disc is either completely are partially removed leaving a space available for the insertion of interbody device 110.

Thereafter, interbody device 110 is connected to inserter/extractor 200. This is achieved by inserting inner member 250 into bore 235 of outer member 210 such that threaded tip 264 extends from insertion end 220 of outer member 210 and such that ball-spring mechanisms 236 engage groove 253. Threaded tip 264 is threaded into threaded opening 119 and bosses 222 are positioned within smooth bores 118. It should be understood that interbody device 110 can be placed onto bosses 222 first and then threaded tip 264 is thereafter threaded to device 110, in which case spring-ball mechanisms 236 engage groove 253 once interbody device 110 is fully seated against concave surface 221. However, threaded tip 264 can thread into threaded opening 119 first after ball-detent mechanism is engaged so that interbody device 110 is drawn onto bosses 222 and into contact with concave surface 221.

Once interbody device 110 is connected to inserter/extractor 200, operator uses inserter/extractor 200 to insert interbody device 110 into the disc space. To assist in insertion, knob 254 of inner member 250 can be impacted at the proximal end thereof via a mallet or the like. Due to the abutment between shoulders 226 and 266 (see FIG. 9G) and connection of knob 254 to outer member 210, the force from the impacts is transferred from inner member 250 to outer member 210 to interbody device 110. In this regard, threads 265 are shielded from stresses imposed by such impacts.

After interbody device 110 is positioned within the disc space, it may be determined that interbody device 110 is not optimally positioned. In this regard, interbody device 110 can then be extracted from the disc space and reinserted. Removal of implant 100 includes aligning indicia 310 of adapter 300 with indicia on outer member 200. This alignment ensures the alignment of the threaded portion 316 of shaft 314 with threaded opening 234 of adapter end 230, thus allowing adapter 300 to be coupled to outer member 210. Adapter 300 is then moved distally such that it slides over knob 254 and adapter end 230 of outer member 200. Knob 254 and adapter end 230 are then located within hollow compartment 308. An inward force is then exerted on knob 304, compressing spring 312, and inserting threaded portion 316 into threaded opening 232, as best shown in FIGs. 10D and 10E. Knob 304 is then rotated to engage threads 234 of threaded opening 232, thereby coupling adapter 300 to outer member 210.

Slap hammer 400 may then be coupled to adapter 300 by inserting threaded projection 402 into threaded opening 324 and engaging the threads therein by rotating guide rod 326. Sliding weight 408 is then moved proximally along guide rod 406 and slammed against proximal bumper 410 to exert a force on system 10 to remove implant 100. Again, due to abutment between shoulders 226 and 266 the force from the impact, however, is redistributed away from inner member 250 and flexible portion 262 and exerted primarily on outer member 210 and adapter 300 thereby protecting flexible portion 262 from damage. In other words, without the shouldering feature (*i.e.,* direct contact between shoulders 226 and 266), the pull force exerted by slap hammer 400 on outer member 210 would serve to move outer member 210 away from the disc space while compression of interbody device 110 within the disc space would provide an opposing force which would be applied to inner member 250 resulting in tension applied to flexible portion 262 of inner member 250 potentially damaging it. However, the shouldering feature transfers forces applied to inner member 250 to outer member 210 before such forces are transferred as tension to flexible member 262. Thus, the shouldering feature shields flexible member 262 from potentially damaging stress during removal of interbody device via slap hammer 400. Adapter 300 also helps shield flexible member 262 from damage by transferring pull forces from slap hammer 400 directly to outer member 210. In other words, if slap hammer 400 were connected to inner member 250 instead of outer member 210 via adapter 300, such as via knob 254, then pull forces from slap hammer 400 would be transferred to inner member and, consequently, to flexible portion 262 potentially damaging the same. Thus, adapter 300 and the shouldering features help protect flexible portion 262 of inner member 262.

Once interbody device 110 is removed from the disc space, slap hammer 400 may be disconnected from adapter 300 by unthreading it from adapter 300. Adapter 300 is removed from outer member 210 by rotating knob 307 counterclockwise to disengage threaded shaft 314 from threaded opening 234 in outer member 210. Spring 312 snaps threaded shaft 314 and knob 304 back to their neutral position after threads of threaded end 316 are fully disengaged which provides feedback to the operator that adapter 300 can then be slid off of the proximal end of inserter/extractor 200. Adapter 300 is then removed from inserter/extractor 200. Thereafter, interbody device 110 can be reinserted back into the disc space in the desired location via impacting inserter/extractor 200 as discussed above.

After interbody device 110 is positioned within the disc space, knob 254 can be rotated to disengage threaded tip 264 of inner member 250 from interbody device 110 and outer member 250 bosses 222 can be pulled clear from smooth bores 118. First inner member 250 is then removed from outer member 210, and second inner member 250' is inserted into outer member 210. Plate assembly 130 is placed adjacent to insertion end 220 of inserter/extractor 200, bosses 222 are positioned within smooth bores 118 of plate 140, threaded tip 264' is inserted into tool opening 179 of connection screw 170, and knob 254 is rotated counterclockwise to thread tip 264' into connection screw 170.

Thereafter, plate assembly 130 is guided to the disc space so that it is positioned adjacent trailing end of interbody device 110. Plate assembly 130 is then connected to interbody device 110 by inserting bosses 146 of plate 140 into smooth bores 118 of interbody device 110 and rotating knob 254 clockwise which rotates connection screw 170 to threadedly engage distal shaft 176 of screw 170 with threaded opening 119. Due to the snap-fit of connection screw 170 to plate 140, plate 140 is drawn toward interbody device 110 and vertebrae so that bone screw openings 150a-b are each positioned adjacent a respective vertebra. Once a certain amount of torque is reached between connection screw 170 and interbody device 110, threads 170 of distal shaft 176 begin to unthread from threaded opening 119 allowing inserter/extractor 200 to be disconnected from plate assembly 130. This is made possible by the right-handed threads of interbody device 110 and threaded end 176 of connection screw 170 and the left-handed threads 265' of second inner member 250' and inner threads 175 of connection screw 170. Once inserter/extractor 200 is fully disconnected from plate assembly 130, a driver instrument (not shown) may then be used to further tighten the connection between plate assembly 130 and interbody device 110. Bone screws 102 are inserted through respective bone screw openings 150a-b. This can be done while plate assembly 130 is connected to inserter/extractor 200 or afterward. Either way, bosses 146 help maintain plate's orientation relative to vertebrae and interbody device 110 during bone screw insertion and tightening of connection screw 170. Once bone screws 102 are fully seated, screw blockers 160 are operated to block bone screws 102 from back-out. This is achieved by engaging a driver to heads 162 of screw blockers 160 and rotating the same to overcome the bias of flexible arm 163 so that screw blockers 160 are rotated from the unblocked position to the blocked position in which lobe 131 of each blocker 160 is positioned over its respective screw head 101.

To summarize the foregoing description, a spinal fusion system comprising: an interbody device having a leading end, trailing end, and opposed bone contacting sides extending therebetween, the trailing end defining a threaded opening extending toward the leading end; a bone plate having inner and outer surfaces and defining a first bone screw opening and connection screw opening extending therethrough; and a connection screw having a head and a threaded shaft, the head being positioned within the connection screw opening of the bone plate and being rotatably connected thereto such that the connection screw is rotatable about a longitudinal axis thereof but is prohibited from translational movement relative to the bone plate, the threaded shaft extending from the connection screw opening and being configured for threaded engagement with threaded opening of interbody device; and/or
the head of the connection screw is snap-fit to the bone plate; and/or
the connection screw opening includes first and second sections thereof, the first section being positioned closer to the outer surface than the second section and having a cross-sectional dimension smaller than the second section such that a first shoulder is formed therebetween, the head of the connection screw being at least partially positioned within the second section of the connection screw opening, the shoulder preventing the connection screw from backing out of the connection screw opening; and/or
the head of the connection screw includes a plurality of flexible members that are movable radially inwardly from a first to a second position when the head is passed through the first section of the connection screw opening and then from the second to the first position when the flexible members are at least partially positioned within the second section; and/or
the flexible members each include a flange extending radially outwardly therefrom; and/or
the threaded shaft includes a right-handed outer thread, and the connection screw defines a threaded interior opening having a left-handed inner thread; and/or
the bone plate includes a second bone screw opening extending therethrough; and/or
the first and second bone screw openings are aligned with the connection screw opening along a longitudinal axis of the bone plate; and/or
the bone plate further defines a screw blocker opening positioned adjacent the bone screw opening, and a screw blocker rotatably positioned within the screw blocker opening; and/or
the screw blocker opening defines first and second grooves extending along a length of the screw blocker opening, and the screw blocker includes a projection that is alternately engageable to the first and second grooves; and/or
the screw blocker includes a flexible arm and the projection extends from the flexible arm, the flexible arm being moveable inwardly when the screw blocker is rotated between the first and second grooves of the screw blocker opening.

Also described was a bone plate assembly comprising: a bone plate having inner and outer surfaces and defining a first bone screw opening and first screw blocker opening, the first bone screw opening extending through the inner and outer surfaces, the first screw blocker opening being positioned adjacent the first bone screw opening and having first and second indentations circumferentially offset from each other about a longitudinal axis of the screw blocker opening; and a first screw blocker positioned within the first screw blocker opening and having a head and a body extending from the head, the body having a projection extending radially outwardly therefrom and being alternately engageable with the first and second indentations of the screw blocker opening; and/or
the first and second indentations are each semi-cylindrical grooves that extend along a portion of a length of the screw blocker opening; and/or
the screw blocker opening includes a first section that extends through the inner surface of the bone plate and is conical and a second section that is cylindrical, the indentations being located within the second section; and/or
the body of the first screw blocker has a post at a distal end thereof, the post having a tool opening defined by a sidewall thereof, the sidewall being radially expanded from a first configuration such that the first screw blocker is rotatable within the first screw blocker opening but is prohibited from translational movement relative to the bone plate; and/or
the screw blocker body includes a flexible arm that depends downwardly from the head and is moveable inwardly as the screw blocker is rotated about a longitudinal axis thereof between the first and second indentations, the projection extending from the flexible arm; and/or
the head of the first screw blocker is asymmetric about a plane extending through the first screw blocker; and/or
the screw blocker opening extends through a recessed portion of the bone plate such that the screw blocker head is positioned within the recessed portion and is moveable within the recess from an unblocked position to a blocked position in which a portion of the head is positioned over the bone screw opening; and/or
further comprising a second screw blocker and wherein the bone plate further defines a second bone screw opening and second screw blocker opening adjacent the first bone screw opening, the second screw blocker being positioned within the second screw blocker opening.

Also described was a method of spinal fusion of adjacent vertebrae of a mammalian subject comprising: connecting an interbody device to a first inner member of an insertion tool positioned within an outer member of the insertion tool; inserting the interbody device into an intervertebral disc space using the insertion tool; disconnecting the interbody device from the first inner member; removing the first inner member from the outer member; inserting a second inner member into the outer member; connecting a bone plate to the second inner member; inserting the bone plate into the mammalian subject adjacent the intervertebral disc space; and driving a bone screw through a bone screw opening of the bone plate and into a first vertebra; and/or
connecting the interbody device to the first inner member includes rotating the first inner member in a first direction, and connecting the bone plate to the second inner member includes rotating the second inner member in a second direction opposite the first direction; and/or
connecting the bone plate to the interbody device while the interbody device is positioned within the disc space; and/or
connecting the bone plate to the interbody device includes rotating the second inner member in the first direction; and/or
connecting the bone plate to the interbody device includes threadedly engaging a connection screw connected to the bone plate to the interbody device; and/or
connecting the bone plate to the second inner member includes threadedly engaging a threaded tip of the second inner member to the connection screw.
connecting an adapter to the outer member, connecting a slap hammer to the adapter, and removing the interbody device from the intervertebral disc space via the slap hammer; and/or
connecting the adapter to the outer member includes sliding the adapter over a knob of the first inner member; and/or
connecting the adapter to the outer member includes pushing and rotating a knob of the adapter so that a threaded shaft of the adapter threadedly engages a threaded opening of the outer member; and/or
the outer member includes an angled shaft and the inner member includes an elongate shaft, a threaded tip, and a flexible portion disposed between the elongate shaft and threaded tip; and/or
inserting the first inner member into a bore of the outer member such that a shoulder between the threaded tip and flexible portion is positioned in a facing relationship with a shoulder within the bore of the outer member; and/or
rotating a screw blocker from a first position to a second position so that a head thereof is positioned over the bone screw.

Also described was a method of spinal fusion of adjacent vertebrae of a mammalian subject comprising: connecting an interbody device to an insertion tool; inserting the interbody device into an intervertebral disc space using the insertion tool; disconnecting the interbody device from the insertion tool; connecting a bone plate assembly to a second inner member, the bone plate assembly having a connection screw rotatably connected thereto such that the connection screw is rotatable about a longitudinal axis thereof but is prohibited from translational movement relative to the bone plate; inserting the bone plate assembly into the mammalian subject adjacent the intervertebral disc space; and threadedly connecting a threaded shaft of the bone plate assembly to the interbody device while the interbody device is positioned within the disc space; and/or
the threadedly connecting step is performed using the insertion tool; and/or
connecting the interbody device to the insertion tool includes rotating a first inner member of the insertion tool in a first direction, and connecting the bone plate assembly to the insertion tool includes rotating a second inner member of the insertion tool in a second direction opposite the first direction; and/or
connecting the bone plate assembly to the interbody device includes rotating the second inner member in the first direction.

Further described was an insertion and extraction system for an interbody device, comprising: an insertion tool having an outer member and an inner member; an adapter having a body defining a hollow compartment therein, the hollow compartment being configured to receive a portion of the outer and inner members, the adapter being connectable to the outer member; and a slap hammer connected to the adapter having a sliding weight and a bumper for being bumped by the sliding weight; and/or
the inner member includes a knob extending from a proximal end of the outer member, the hollow compartment being configured to receive the proximal end of the outer member and the knob; and/or
the adapter includes a threaded opening extending therein, and the slap hammer has a threaded projection engageable with the threaded opening of the adapter; and/or
the adapter includes a spring and threaded shaft, the spring and threaded shaft being disposed within a transverse opening extending into the body of the adapter and communicating with the hollow compartment, the spring and threaded shaft being arranged such that the spring biases a threaded end of the threaded shaft away from the hollow compartment; and/or
the body of the adapter includes a post and the transverse opening extends through the post, and the adapter includes a knob disposed over a portion of the post and is connected to the inner shaft such that rotating the knob rotates the threaded shaft; and/or
the threaded shaft includes a collar extending radially outwardly therefrom and the spring is arranged such that its biases pushes against the collar; and/or
pushing on the knob overcomes the bias of the spring to translate the threaded shaft from a first position in which the threaded end is disposed within the post and a second position in which the threaded tip is positioned within the hollow cavity; and/or
the outer member defines a transverse threaded opening extending therein; and/or
when the insertion tool is received within the hollow cavity of the adapter, the threaded end of the threaded shaft aligns with the transverse threaded opening of the outer member so that threaded member can be moved into engagement with the threaded opening over the bias of the spring.

Even further described was a method of extracting an interbody device from an intervertebral disc space comprising the steps of: sliding an adapter over a handle of an inserter; actuating a knob of the adapter to dispose a shaft of the adapter within a bore of the inserter; coupling a slap hammer to the adapter, the slap hammer including a sliding weight and a guide rod; and sliding the sliding weight along the guide rod to exert a force on the handle in a direction opposite the implant so as to transfer such force to the implant to remove it from the intervertebral disc space; and/or
connecting the adapter to the inserter by threadedly engaging a threaded shaft of the adapter with the bore of the inserter which is a threaded bore; and/or
actuating the knob of the adapter further comprises pushing on the knob to overcome a spring biasing the threaded shaft and turning the knob to threadedly engage a threaded end of the threaded shaft and the bore; and/or
the inserter includes an outer member and an inner member, the inner member having a threaded tip, an elongate shaft, and a flexible portion disposed between the threaded tip and elongate shaft; and/or
the outer member includes the handle and sliding the adapter over the handle of the inserter also includes sliding the adapter over a knob of the inner member; and/or
the slap hammer further comprises a threaded projection extending from a distal end surface and along a longitudinal axis of the slap hammer, and coupling the slap hammer to the adapter further comprises threading the threaded projection into a threaded bore at a proximal surface of the adapter, the threaded bore extending into the adapter along a longitudinal axis thereof; and/or
aligning indicia on the adapter with indicia on the inserter prior to sliding the adapter onto the inserter.

## Claims

1. A spinal fusion system comprising:
an interbody device having a leading end, trailing end, and opposed bone contacting sides extending therebetween, the trailing end defining a threaded opening extending toward the leading end;
a bone plate having inner and outer surfaces and defining a first bone screw opening and connection screw opening extending therethrough; and
a connection screw having a head and a threaded shaft, the head being positioned within the connection screw opening of the bone plate and being rotatably connected thereto such that the connection screw is rotatable about a longitudinal axis thereof but is prohibited from translational movement relative to the bone plate, the threaded shaft extending from the connection screw opening and being configured for threaded engagement with threaded opening of interbody device.

2. The system of claim 1, wherein the head of the connection screw is snap-fit to the bone plate.

3. The system as in claim 1 or 2, wherein the connection screw opening includes first and second sections thereof, the first section being positioned closer to the outer surface than the second section and having a cross-sectional dimension smaller than the second section such that a first shoulder is formed therebetween, the head of the connection screw being at least partially positioned within the second section of the connection screw opening, the shoulder preventing the connection screw from backing out of the connection screw opening.

4. The system of claim 3, wherein the head of the connection screw includes a plurality of flexible members that are movable radially inwardly from a first to a second position when the head is passed through the first section of the connection screw opening and then from the second to the first position when the flexible members are at least partially positioned within the second section.

5. The system of claim 4, wherein the flexible members each include a flange extending radially outwardly therefrom.

6. The system as in any of the preceding claims, wherein the threaded shaft includes a right-handed outer thread, and the connection screw defines a threaded interior opening having a left-handed inner thread.

7. The system as in any of the preceding claims, wherein the bone plate includes a second bone screw opening extending therethrough.

8. The system of claim 7, wherein the first and second bone screw openings are aligned with the connection screw opening along a longitudinal axis of the bone plate.

9. The system as in any of the preceding claims, wherein the bone plate further defines a screw blocker opening positioned adjacent the bone screw opening, and a screw blocker rotatably positioned within the screw blocker opening.

10. The system of claim 9, wherein the screw blocker opening defines first and second grooves extending along a length of the screw blocker opening, and the screw blocker includes a projection that is alternately engageable to the first and second grooves.

11. The system of claim 10, wherein the screw blocker includes a flexible arm and the projection extends from the flexible arm, the flexible arm being moveable inwardly when the screw blocker is rotated between the first and second grooves of the screw blocker opening.

12. The system as in any of the preceding claims, wherein the interbody device includes a porous structure that forms at least a portion of the opposed bone contacting sides.

13. The system of claim 12, wherein the interbody device includes vertical and horizontal graft windows extending therein and the porous structure defines at least a portion of a perimeter of the vertical and horizontal graft windows.

14. The system of claim 13, wherein the porous structure has an average pore diameter of 100 to 1000 microns with a 30% to 80% porosity.

15. The system of claim 13, wherein the interbody device includes lateral sidewalls, the lateral sidewalls, leading end, and trailing end being made from a solid material.
